# EUROPEAN PATENT APPLICATION

(11) **EP 2 371 420 A1**
(43) Date of publication of application: **05.10.2011**
(21) Application number: 11171701.3
(22) Date of filing: 14.05.2007
(51) Int. Cl.: A61P 25/04, A61P 29/00, A61K 31/137, A61K 31/405, A61K 31/5415, A61P 19/02

(54) **Veterinary pharmaceutical compositions for the treatment of pain and inflammation**

(30) Priority: 18.05.2006 IT MI20060983
(62) Divisional of application: 07734560.1
(71) Applicant: Formevet S.p.A., 20149 Milano (IT)
(72) Inventor: FORMENTI, Andrea, 20149 MILANO (IT); FORMENTI, Filippo, 20149 MILANO (IT)
(74) Representative: Minoja, Fabrizio

(57) **Abstract**

This invention relates to veterinary pharmaceutical compositions based on a combination of tramadol and meloxicam or carprofen for the treatment of pain and inflammation in animals.

## Description

### FIELD OF INVENTION

The present invention relates to veterinary pharmaceutical compositions for the treatment of pain and inflammation, containing a combination of:
a) tramadol, and
b) meloxicam or carprofen.

### INTRODUCTION

The increasing integration and coexistence of dogs and cats in the human residential and social context has led humans to treat these animals almost as members of the family, devoting special care and attention to their diet, treatment and other needs.

The result is that the average life span of dogs and cats has greatly increased and, as in the case of humans, the health problems associated with old age have multiplied.

Many degenerative diseases typical of elderly animals involve pain, often accompanied by inflammatory processes.

Moreover, increasingly strict breeding criteria have led to the appearance of degenerative diseases, even in young dogs. This applies to hip dysplasia, a hereditary disorder that affects the coxofemoral joint, and eventually causes discomfort and pain. The affected animals feel pain in their hind limbs, leading to a reduction in physical activity, difficulty in getting up, walking upstairs, running, etc..

In cats, the arthritis which appears with old age is often aggravated by obesity, which is becoming increasingly common because of the sedentary lifestyle and often the intrinsic greed of these animals. Moreover, arthritis is more difficult to treat in cats, which are more sensitive to the usual anti-inflammatories and often liable to kidney failure when elderly.

Effective treatment of pain and inflammation would considerably improve the animal's quality of life, especially in the case of elderly animals.

A specific characteristic of elderly dogs is the difficulty of administering medicines, due to probable and existing dental problems, lack of appetite, and the often unpleasant flavour of medicinal products.

It is even more difficult to administer medicinal products to cats of all ages in view of their innate suspicion, often accompanied by strong dislike of food with an unusual or unpleasant odour.

Horses, especially racehorses, are particularly subject to problems of the musculoskeletal apparatus, which undergoes great stresses, resulting in traumas, microtraumas, chip fractures, and degeneration of the joint cartilage.

Osteoarthritis in particular is one of the major causes of lameness. In addition to a form of primary osteoarthritis, in which inflammation and degeneration take place, especially in the joints subjected to greatest wear as a result of heavy loads (the intervertebral cervical and lumbar, metacarpo-phalangeal, interphalangeal, stifle and ankle joints), a form of secondary osteoarthritis due to specific causes such as osteochondrosis, navicular disease, fatigue or stress fractures may also be present. Problems with the tendons and ligaments of the joints may also arise.

Other inflammatory forms common in horses are navicular disease (palmar hoof inflammation), ringbone (osteoarthritis of the pastern and coffin joint), and inflammation due to diseases such as splint, spavin, laminitis and wind gall.

### PRIOR ART

Meloxicam and carprofen belong to the category of non-steroidal anti-inflammatory drugs (NSAIDs). These substances have long been used in large amounts in veterinary medicine due to their anti-inflammatory, analgesic and antipyretic action. However, their analgesic action is considered to be limited, and their isolated administration is only recommended for the treatment of mild to moderate pain.

NSAIDs are "peripheral" analgesics because they act on the peripheral nervous system, and directly treat the inflammation responsible for sensitisation of the nociceptors.

The action mechanism of NSAIDs involves inhibition of arachidonic acid catabolism, and consequently of prostaglandin (PG) synthesis, the prostaglandins being the main mediators of inflammation and peripheral sensitisation of the nociceptors. However, inhibition of prostaglandin (PG) synthesis also causes numerous side effects of NSAIDs on the digestive apparatus, the haemopoietic system and the kidneys, which make their use inadvisable in medium/long-term treatments.

As regards pharmacokinetics, NSAIDs present rapid, complete absorption, whether they are given by oral or parenteral administration, and a modest distribution volume (approximately 10%) due to the bond with the serum proteins, and are mainly eliminated through the urine.

Food can reduce the oral absorption of some NSAIDs, or cause other pharmacological interactions.

Injectable preparations tend to be alkaline, and can cause necrosis or pain if deposited in the perivascular area.

There are also considerable differences in the rate of elimination between different animal species.

Tramadol is a central analgesic with a dual action mechanism: it is a pure non-selective agonist of the µ, δ and κ opiate receptors, with a greater affinity for the µ receptors, and is a modulator of the noradrenergic and serotoninergic transmission of pain and inflammation at central synaptic level. Tramadol consequently acts on both systems which inhibit pain and inflammation: the opioid and monoaminergic systems.

Tramadol combines an effective, rapid analgesic action with an excellent global tolerability profile. Unlike the other central analgesics, such as morphine, when administered at therapeutic doses it does not cause respiratory depression, possesses good cardiovascular tolerability, and does not affect gastrointestinal motility. Consequently, tramadol is a first-choice drug for the treatment of acute and chronic moderate or severe pain of different types and causes.

It is important to note that the analgesic efficacy of tramadol after oral administration is not significantly different from that recorded after parenteral administration.

Pharmacokinetic studies of tramadol, conducted on 6 animal species (mouse, hamster, rat, guinea pig, rabbit and dog) have demonstrated:
- excellent absorption, regardless of the formulation used. When administered by the i.m. route, absorption is almost total;
- peak plasma concentration within 2 hours;
- a modest bond with the plasma proteins;
- elimination mainly by the renal route, with a half-life of approx. 2.5 hours in the dog, regardless of the administration route.

NSAIDs are the drugs most often used to treat pain and inflammation in animals.

P. Vasseur et al., JAVMA, 20, 807 (1995) describe the use of carprofen in the treatment of osteoarthritis in the dog.

D. Knapp et al., J. Vet. Int. Med., 8, 27 (199) describe the use of piroxicam in the treatment of pain in dogs suffering from carcinoma.

### DESCRIPTION OF THE INVENTION

It has now been found that the combination of tramadol and meloxicam or carprofen is particularly effective in the treatment of pain and inflammation in domestic animals.

This invention therefore relates to veterinary pharmaceutical compositions based on an combination of tramadol and meloxicam or carprofen for the treatment of pain and inflammation in domestic animals.

More particularly, this invention relates to compositions based on a combination of tramadol and meloxicam or carprofen for the treatment of pain and inflammation in pets, such as dogs, cats and horses.

The effect of the combination according to the invention, namely tramadol (an analgesic with a central action) and meloxicam or carprofen (anti-inflammatories with a peripheral action) is greater than the sum of the effects obtained after separate administration of the individual components of the combination, due to synergy between the components of the combination. This synergic effect provides an extremely effective analgesic action and enables the usual dose of NSAIDs to be reduced, thus reducing their side effects.

The compositions according to the invention therefore allow more effective, safer anti-inflammatory and painkilling treatment which can be performed over the medium/long term, and present greater efficacy and tolerability than drugs consisting of only one of the two active constituents, with obvious therapeutic advantages, better analgesic results, and limited side effects.

The combination according to the invention will be used to treat painful or inflammatory conditions, such as inflammation of the musculoskeletal system, inflammation of the soft or hard tissues, joint diseases, arthritis, osteoarthritis, gouty arthritis, myositis, laminitis, the sequelae of trauma, pain of any origin, including pain caused by cancer, mastitis and equine colic, in pets, especially dogs, cats and horses.

The combination according to the invention is particularly useful in intramuscular diseases of elderly animals or in the event of concomitant pathological states such as kidney failure, gastroenteric disorders, and haemostasis alterations.

The compositions according to the invention can be formulated in a way suitable for oral or parenteral administration to animals, and can be prepared according to conventional methods well known in pharmaceutical technology, such as those described in "Remington's Pharmaceutical Handbook", Mack Publishing Co., N.Y., USA, using excipients, diluents, filling agents and anti-caking agents acceptable for their final use.

Examples of compositions for oral administration are water-dispersible tablets, palatable tablets, palatable boli, oral gels or pastes, and drops.

Examples of compositions for the parenteral administration are injectable solutions.

The oral compositions will contain the active ingredients in the following amounts per unit dose:
a) tramadol between approx. 25 and approx. 50 mg; and
b) meloxicam between approx. 2.5 and 2.5 mg; or carprofen between approx. 10 and approx. 50 mg.

The parenteral compositions will contain the active ingredients in the following amount per unit dose (1 ml ampoule):
a) tramadol between approx. 25 and approx. 50 mg; and
b) meloxicam between approx. 1 and approx. 5 mg; or carprofen between approx. 25 and approx. 50 mg.

The compositions according to the invention must be administered in such a way as to provide the following doses of the active constituents:
a) tramadol: 1 to 4 mg/kg of body weight; and
b) meloxicam: 2.5 to 2.5 mg/kg of body weight; or carprofen: 2.5 to 2 mg/kg of body weight.

Examples of compositions according to the invention are set out below.

### EXAMPLE 1 - TABLETS

| Ingredients | Amount (mg) |
|---|---|
| Tramadol | 35 |
| Meloxicam | 0,070 |
| Microcrystalline cellulose | 230 |
| Corn starch | 50 |
| Sodium saccharinate | 10 |
| Magnesium stearate | 2 |
| Anhydrous colloidal silicon dioxide | 2 |
| Flavouring agents | 15 |

### EXAMPLE 2 - TABLETS

| Ingredients | Amount (mg) |
|---|---|
| Tramadol | 40 |
| Carprofen | 35 |
| Microcrystalline cellulose | 221 |
| Corn starch | 50 |
| Sodium saccharinate | 10 |
| Magnesium stearate | 2 |
| Anhydrous colloidal silicon dioxide | 2 |
| Flavouring agents | 15 |

### EXAMPLE 3 - AMPOULES

| Ingredients | Amount (mg) |
|---|---|
| Tramadol | 30 |
| Meloxicam | 3,5 |
| Sodium acetate | 4,15 |
| Water for injection | 951 |

### EXAMPLE 4 - AMPOULES

| Ingredients | Amount (mg) |
|---|---|
| Tramadol | 40 |
| Carprofen | 35 |
| Sodium acetate | 4,15 |
| Water for injection | 951 |

## Claims

1. Veterinary pharmaceutical compositions for the treatment of pain and inflammation, containing a combination of:
a) tramadol, and
b) meloxicam or carprofen.

2. Compositions as claimed in claim 1, for the oral administration, containing the active ingredients in the following amounts per unit dose:
a) tramadol between approx. 25 and approx. 50 mg; and
b) meloxicam between approx. 2.5 and 2.5 mg; or carprofen between approx. 10 and approx. 50 mg.

3. Compositions as claimed in claim 1, for the parenteral administration, containing the active ingredients in the following amounts per unit dose:
a) tramadol between approx. 25 and approx. 50 mg; and
b) meloxicam between approx. 1 and approx. 5 mg; or carprofen between approx. 25 and approx. 50 mg.

4. Use of a combination of tramadol and meloxicam or carprofen for the preparation of a medicament for the veterinary use for the treatment of pain and inflammation in animals.

5. Use as claimed in claim 4, wherein the animals are pets.

6. Use as claimed in claim 5, wherein the pets are dogs, cats and horses.
